# FASCICULE DE BREVET EUROPEEN

(11) **EP 3 389 629 B1**
(45) Date de publication et mention de la délivrance du brevet: **21.04.2021**
(21) Numéro de dépôt: 15834751.8
(22) Date de dépôt: 17.12.2015
(51) Int. Cl.: A61K 9/00, A61K 35/644, A61P 39/00, A61K 47/36, A61K 9/08, A61K 47/26

(54) **COMPOSITION LIQUIDE POUR SON UTILISATION COMME SOLUTION NASALE**
FLÜSSIGZUSAMMENSETZUNG ZUR VERWENDUNG ALS NASALE LÖSUNG
LIQUID COMPOSITION FOR USE AS A NASAL SOLUTION

(43) Date de publication de la demande: 24.10.2018
(73) Titulaire: Urgo Recherche Innovation et Développement, 21300 Chenove (FR); Hcp Healthcare Asia Pte.Ltd, Singapore 138623 (SG)
(72) Inventeur: DERAIN, Nathalie, 21370 Prenois (FR)
(74) Mandataire: Hurtiger, Myriam
(86) Numéro de dépôt international: PCT/FR2015/053592
(87) Numéro de publication internationale: WO 2017/103348

(56) Documents cités:
- EP-A1- 1 063 885
- WO-A1-2014/158119
- ES-A1- 2 190 730
- US-A1- 2005 118 278
- US-A1- 2007 158 269
- US-A1- 2008 274 163
- US-A1- 2008 275 030
- US-B2- 6 641 799
- DATABASE GNPD [Online] MINTEL; 28 August 2015 (2015-08-28), anonymous: "Apple Flavour Infant Syrup with Glycerol", XP55607180, retrieved from www.gnpd.com Database accession no. 3401199

## Description

La présente invention a pour objet une composition liquide comprenant au moins un agent tapissant des muqueuses, au moins un humectant et au moins un ajusteur de pH pour son utilisation comme solution nasale dans la prévention et le traitement des pathologies hivernales, en particulier les affections des voies respiratoires supérieures.

La solution nasale selon la présente invention permet de tapisser les cavités nasales ainsi que le rhinopharynx. Ceci permet de limiter l'adhésion de bactéries et/ou le passage de virus. Cette limitation se traduit par une diminution des affections des voies respiratoires supérieures (telles que par exemple les sinusites ou otites) ainsi que des symptômes liés à ces affections.

Il existe un besoin pour de nouvelles compositions présentant une bonne tolérance par les muqueuses, capables de les tapisser in situ et rapidement, présentant une texture optimale permettant une couverture maximale et uniforme de la zone à traiter, à savoir les cavités nasales et le rhinopharynx, et présentant une acidité et une viscosité stable dans le temps.

La Demanderesse a trouvé, de manière surprenante, qu'il était possible de tapisser les muqueuses et de limiter l'adhésion de bactéries et/ou la pénétration de virus au moyen d'une composition liquide particulière. La Demanderesse a également trouvé de manière inattendue qu'en diminuant et maintenant le pH entre 3,3 et 4, de préférence entre 3,5 et 3,7, l'action sur les bactéries et/ou les virus était potentialisée.

L'invention a ainsi pour objet, selon un premier aspect, une composition liquide comprenant au moins un agent tapissant des muqueuses présent en une teneur allant de 5% à 25% en poids, de préférence allant de 10 à 20% en poids, par rapport au poids total de la composition, au moins un humectant et au moins un ajusteur de pH.

Les compositions telles que les solutions nasales sont connues de l'homme du métier. Le brevet EP 1 063 885 décrit par exemple d'associer du xylitol et/ou du xylose et une solution saline pour son utilisation dans le traitement par administration nasale d'infections du nasopharynx. Cette composition présente notamment l'avantage de nettoyer l'oropharynx et de réduire les populations de bactéries pathogènes. Toutefois, cette solution, de par sa composition, ne reste localement que brièvement et ne permet pas de tapisser les muqueuses. Ainsi, la composition décrite ne limite pas la pénétration de virus qui peuvent également être à l'origine d'affections des voies respiratoires supérieurs. Son action est donc limitée dans le temps et dans le type d'infections (infections bactériennes).

L'invention se rapporte également à l'utilisation de la composition comme solution nasale dans la prévention et le traitement des pathologies hivernales, en particulier les affections des voies respiratoires supérieures.

En particulier, la composition liquide selon l'invention est physiologiquement acceptable, c'est à dire non toxique et susceptible d'être appliquée sur les tissus tels que les muqueuses d'êtres humains ou d'animaux et permet la formation d'un tapissage biocompatible. Cette composition, compte tenu de son application, ne doit contenir que des ingrédients alimentaires.

On entend par composition liquide, au sens de la présente demande, une composition qui s'écoule sous son propre poids.

### Agent tapissant des muqueuses

La composition selon l'invention comprend au moins un agent tapissant des muqueuses. Par agent tapissant des muqueuses on entend tout composé capable de rentrer et rester en contact avec la muqueuse de manière durable (pendant au moins une heure).

De manière préférentielle, cet agent peut, de préférence, également masquer l'acidité de l'ajusteur de pH contenu dans la composition.

L'agent tapissant des muqueuses est choisi parmi les polyols, tel qu'un sirop de maltitol, isomaltitol, mannitol, isosorbide, glycol, érythritol, arabitol, ribitol, dulcitol, volemitol ou lactitol ou une combinaison de ces ingrédients.

Compte-tenu de son application, l'agent tapissant des muqueuses doit être alimentaire.

De préférence, l'agent tapissant est un sirop de maltitol.

L'agent tapissant des muqueuses est présent en une teneur allant de 5% à 25% en poids, de préférence allant de 10 à 20% en poids, par rapport au poids total de la composition.

### Humectant

La composition selon l'invention comprend au moins un humectant.

Par humectant on entend toute substance qui a tendance à absorber l'humidité de l'air, par absorption ou par adsorption. De manière générale, l'humectant est capable de retenir l'eau et de maintenir l'hydratation.

L'humectant selon la présente invention est choisi parmi le chlorure de sodium, l'acétate de calcium, la glycérine, le propylène glycol, le miel, la carboxymethylcellulose sodique, l'oxyde de magnésium, le sorbitol, le chlorure de calcium, le fructose, le glucose, le maltose, le mannose, les polyethylène glycol tels que, par exemple, PEG 200, PEG 300, PEG 400, PEG 600, la triacetine, le sodium malate, l'acide lactique, les phosphates.

De préférence, l'humectant est la glycérine.

L'humectant est présent en une teneur allant de 0,1% à 5% en poids, de préférence allant de 0,5% à 2% en poids, par rapport au poids total de la composition.

### Ajusteur de pH

L'ajusteur de pH utilisé dans la présente invention est un couple acide/base conjuguée, permettant d'apporter à la solution un pouvoir tampon. Celui-ci est choisi de manière à maintenir le pH de la solution entre 3,3 et 4, de préférence entre 3,5 et 3,7 de manière durable et stable dans le temps.

L'ajusteur de pH est choisi parmi les couples acide citrique/citrate, acide phosphorique/phosphate, acide tartrique/tartrate, acide malique/malate, acide sorbique/sorbate,

L'ajusteur de pH est présent en une teneur nécessaire pour avoir un effet tampon.

Dans le cadre de la présente invention, la combinaison de citrate trisodique dihydraté et d'acide citrique s'est avéré être le meilleur ajusteur de pH. Cette combinaison permet de maintenir le pH entre 3,5 et 3,7 pendant au moins 24 mois après la première utilisation lorsque la composition est conditionnée dans un flacon.

Le pH est également maintenu entre 3,5 et 3,7 plusieurs heures lorsque la composition est appliquée sur les muqueuses.

### Milieu pharmaceutiquement acceptable

La composition selon l'invention comprend un milieu pharmaceutiquement acceptable comprenant au moins un solvant.

Par milieu pharmaceutiquement acceptable, on entend, au sens de la présente demande, un milieu compatible avec la muqueuse.

Tout solvant compatible avec l'agent tapissant peut être utilisé, de préférence on utilisera l'eau.

Le solvant peut représenter de 70 à 90% en poids, de préférence de 80 à 85% en poids, par rapport au poids total de la composition.

### Additifs

La composition selon l'invention peut comprendre un ou plusieurs additifs pharmaceutiquement acceptables, comme par exemple les parfums, les arômes, les colorants, les pigments, les agents rhéologiques, les conservateurs, les vitamines, les huiles essentielles et les agents actifs, notamment choisis parmi les agents anti-bactériens, les antiseptiques, les anti-viraux, les agents antifongiques, les anti-douleurs, les anti-inflammatoires, les agents hydratants, les actifs restructurants et les anesthésiques.

Par « conservateur », on entend tout produit capable de protéger la composition contre toute contamination microbienne, que ce soit en cours de production ou durant l'utilisation de la composition.

Le conservateur est choisi parmi les conservateurs alimentaires tels que, par exemple, l'acide sorbique, le sorbate de sodium, le sorbate de potassium, le sorbate de calcium, le parahydroxybenzoate d'hepthyle, l'acide benzoïque, le benzoate de sodium, le benzoate de potassium, le benzoate de calcium, le chlorure de benzalkonium, l'acide formique, le formiate de sodium, le formiate de calcium, le formaldéhyde, l'acide acétique, les acétates de potassium, l'acétate de sodium, le diacétate de sodium, l'acétate de calcium, l'acétate d'ammonium, l'acide déhydroacétique, le déhydroacétate de sodium, l'acide lactique, l'acide propanoïque, l'acide borique, l'acide malique, l'acide fumarique ou une combinaison de ces conservateurs.

De préférence, le conservateur utilisé dans le cadre de la présente invention est le chlorure de benzalkonium.

Par « arômes », on entend des composés d'origine naturelle ou synthétique susceptibles à eux seuls de modifier le goût de la composition. Des exemples d'arômes sont l'abricot, l'ananas, la banane, le cassis, la cerise, le citron, le lime, la fraise, la framboise, les fruits de la passion, la mandarine, la menthe, l'orange, la pistache, la poire, le réglisse, la rose, l'anis, la myrtille, la cannelle, le gingembre, le pamplemousse, la pomme, le kiwi, le lychee, le melon, la noix de coco, la pêche, la groseille, la goyave, la grenadine, la mangue, la mûre, la papaye, la vanilline, l'extrait naturel de vanille, les huiles essentielles et leurs mélanges.

De préférence, l'arôme utilisé dans la présente invention pour masquer le goût est la menthe.

Par ailleurs, les actifs pouvant être introduits dans la composition selon l'invention peuvent être choisis parmi :
- les anti-bactériens tels que le Polymyxine B, les pénicillines (Amoxycilline), l'acide clavulanique, les tétracyclines, la Minocycline, la chlorotétracycline, les aminoglycosides, l'Amikacine, la Gentamicine, la Néomycine, l'argent et ses sels (Sulfadiazine argentique), les probiotiques ;
- les antiseptiques tels que le mercurothiolate de sodium, l'éosine, la chlorhexidine, le borate de phénylmercure, l'eau oxygénée, la liqueur de Dakin, le triclosan, le biguanide, l'hexamidine, le thymol, le Lugol, la Povidone iodée, le Merbromine, le Chlorure de Benzalkonium et de Benzethonium, l'éthanol, l'isopropanol ;
- les anti-viraux tels que l'Aciclovir, le Famciclovir, le Ritonavir ;
- les antifongiques tels que les polyènes, le Nystatin, l'Amphotéricine B, la Natamycine, les imidazolés (Miconazole, Ketoconazole, Clotrimazole, Éconazole, Bifonazole, Butoconazole, Fenticonazole, Isoconazole, Oxiconazole, Sertaconazole, Sulconazole, Thiabendazole, Tioconazole), les triazolés (Fluconazole, Itraconazole, Ravuconazole, Posaconazole, Voriconazole), les allylamines, la Terbinafine, l'Amorolfine, la Naftifine, la Buténafine ;
- la Flucytosine (antimétabolite), la Griséofulvine, la Caspofungine, la Micafungine ;
- les anti-douleurs tels que le Paracétamol, la Codéine, le Dextropropoxyphène, le Tramadol, la Morphine et ses dérivés, les Corticoïdes et dérivés ;
- les anti-inflammatoires tels que les Glucocorticoïdes, les anti inflammatoires non stéroïdiens, l'Aspirine, l'Ibuprofène, le Kétoprofène, le Flurbiprofène, le Diclofénac, l'Acéclofénac, le Kétorolac, le Méloxicam, le Piroxicam, le Ténoxicam, le Naproxène, l'Indométacine, le Naproxcinod, le Nimésulide, le Célécoxib, l'Etoricoxib, le Parécoxib, le Rofécoxib, le Valdécoxib, la Phénylbutazone, l'acide niflumique, l'acide méfénamique ;
- les agents hydratants tels que l'acide hyaluronique, l'urée, les acides gras, modulateurs des aquaporines, les huiles végétales, le chitosan, les beurres et les cires ;
- les actifs restructurants tels que la vitamine E, la camomille, l'extrait de prêle, le Lipester de soie;
- les anesthésiques tels que la benzocaïne, la lidocaïne, la dibucaïne, le chlorhydrate de pramoxine, la bupivacaïne, la mepivacaïne, la prilocaïne, l'étidocaïne ;

### Utilisation de la composition

L'invention a pour objet l'utilisation de la composition telle que définie précédemment comme solution nasale dans la prévention et le traitement des pathologies hivernales, en particulier les affections des voies respiratoires supérieures, ainsi que les symptômes liés à ces affections.

Par « affection des voies respiratoires supérieures » on entend au sens de la présente invention des affections dues à une infection virale ou bactérienne touchant les voies respiratoires supérieures, soit le nez, les sinus para-nasaux, le pharynx, le larynx et l'oreille moyenne.

Ces affections peuvent être classées en fonction du symptôme prédominant: angine, laryngite, mal de gorge, pharyngite, rhinite, rhino-sinusite, sinusite, toux ou otite

La composition selon l'invention présente une tolérance fonctionnelle excellente lorsqu'elle est appliquée sur des sujets pendant une durée prolongée, et permet de prévenir et traiter les affections des voies respiratoires supérieures ainsi que les symptômes liés à ces affections.

La composition selon l'invention peut être conditionnée en flacon pompe permettant permettant de sprayer la composition.

La présente invention est illustrée plus en détails dans les exemples non limitatifs décrits ci-après.

### Exemple 1 : composition selon l'invention (Tableau 1)

On a préparé une composition selon l'invention ayant la composition suivante :

| Composition | % en poids |
|---|---|
| Acide citrique commercialisé par la société DSM | 0,83 |
| Citrate trisodique dihydraté commercialisé par la société Jungbunzlauer | 0,52 |
| Maltitol commercialisé par la société Roquette sous la dénomination commerciale Lycasin® 80/55 | 15,00 |
| Glycérine 4810 (glycerol) commercialisée par la société Oleon | 1,00 |
| Arôme de menthe commercialisé par la société IFF | 0,40 |
| Chlorure de Benzalkonium (solution 50%) commercialisé par la société Lonza | 0,04 |
| Eau purifiée | 82.21 |

On pèse l'eau et on y ajoute le citrate trisodique dihydraté puis l'acide citrique. Le tout est homogénéisé pendant 10 minutes à l'aide d'un agitateur magnétique à faible agitation. Le pH de cette solution doit être compris entre 3,5 et 3,7. Le sirop de maltitol, la glycérine, l'arôme de menthe et le chlorure de benzalkonium sont ensuite ajoutés à cette solution et le tout est homogénéisé durant 10 minutes à l'agitateur à faible agitation.

La solution ainsi obtenue est alors mise en flacons.

### Exemple 2 : activité virucide de la composition selon l'exemple 1.

La capacité virucide selon la norme française NF T 72-180 (décembre 1989) ou EN 14476 (janvier 2007) a permis de démontrer l'activité de la solution selon l'invention.

La détermination de l'activité virucide de la solution selon l'exemple 1 a été évaluée avec les virus suivants :
- Parainfluenza 3 humain (Para 3)
- Influenza virus A-H1N1 (A-H1N1)
- Rhinovirus équin (ERV) utilisé comme « virus modèle » du Rhinovirus humain
- Pseudorage porcine (PRV), virus de la maladie d'Aujeszky)

Trois temps de contact (10, 20 et 30 minutes) ont été mesurés. Les expériences ont été réalisées à 32°C±1 °C.

Le produit selon l'exemple 1 est testé, pour prouver son efficacité doit diminuer le titre viral de 4 log.
R : Réduction du titre viral en log

Les résultats des tests sont les suivants :

**Tableau 2 : résultats du test selon l'exemple 2**

| Produit testé | Virus testé | Concentration du produit selon l'exemple 1 | Norme utilisée | Temps (minutes) | Réduction du titre viral |
|---|---|---|---|---|---|
| | Para 3 | 80% | NF T 72-180 | 10 | R>5.83 |
| | | 80% | | 20 | R>5.83 |
| | | 80% | | 30 | R>5.83 |
| | Para 3 | 80% | EN 14476 | 10 | R=5 |
| | | 80% | | 20 | R>5.26 |
| | | 80% | | 30 | R>5.26 |
| | ERV | 80% | EN 14476 | 10 | R>5.01 |
| | | 80% | | 20 | R>4.38 |
| Solution selon l'exemple 1 | | 80% | | 30 | R>4.38 |
| | ERV | 80% | NF T 72-180 | 10 | R>4.89 |
| | | 80% | | 20 | R>4.89 |
| | | 80% | | 30 | R>4.89 |
| | PRV | | NF T 72-180 | 10 | R>5.72 |
| | | | | 20 | R>5.72 |
| | | | | 30 | R>5.72 |
| | A-H1N1 | 80% | EN 14476 | 1 | R>4 |
| | | 40% | | 1 | R>4 |
| | | 20% | | 1 | R>4 |

Ces tests ont permis de démontrer l'activité vrucide de la composition selon l'invention vis-à-vis des virus parainfluenza 3 humain, influenza virus A-H1N1, Rhinovirus équin et pseudorage porcine (PRV).

## Revendications

1. Composition destinée à être appliquée sur la muqueuse pour son utilisation comme solution nasale dans la prévention et le traitement des pathologies hivernales, en particulier les affections des voies respiratoires supérieures comprenant au moins un agent tapissant des muqueuses choisi parmi le sirop de maltitol, isomaltitol, mannitol, isosorbide, glycol, érythritol, arabitol, ribitol, dulcitol, volemitol ou lactitol ou une combinaison de ces ingrédients, au moins un humectant choisi parmi le chlorure de sodium, l'acétate de calcium, la glycérine, le propylène glycol, le miel, la carboxymethylcellulose sodique, l'oxyde de magnésium, le sorbitol, le chlorure de calcium, le fructose, le glucose, le maltose, le mannose, les polyethylène glycol, la triacetine, le sodium malate, l'acide lactique, les phosphates et au moins un ajusteur de pH, la dite composition présente un pH compris entre 3,3 et 4, de préférence entre 3,5 et 3,7.

2. Composition pour utilisation selon la revendication 1, **caractérisée en ce que** la teneur totale en agent tapissant des muqueuses est présent en une teneur allant de 5% à 25% en poids, de préférence allant de 10 à 20% en poids, par rapport au poids total de la composition.

3. Composition pour utilisation selon l'une des revendications 1 à 2, **caractérisée en ce que** l'agent tapissant des muqueuses est un sirop de maltitol.

4. Composition pour utilisation selon l'une des revendications 1 à 3, **caractérisée en ce que** l'humectant est présent en une teneur allant de 0,1% à 5% en poids, de préférence allant de 0,5% à 2% en poids, par rapport au poids total de la composition.

5. Composition pour utilisation selon l'une des revendications 1 à 4, **caractérisée en ce que** l'humectant est la glycérine.

6. Composition pour utilisation selon l'une des revendications 1 à 5, **caractérisée en ce que** l'ajusteur de pH est choisi parmi les couples acide citrique/citrate, acide phosphorique/phosphate, acide tartrique/tartrate, acide malique/malate, acide sorbique/sorbate, de préférence une combinaison d'acide citrique et de citrate trisodique dihydraté.

7. Composition pour utilisation selon l'une des revendications 1 à 6, **caractérisée en ce qu'**elle comprend en outre au moins un conservateur et au moins un arôme.

8. Composition pour utilisation selon l'une des revendications précédentes, **caractérisée en ce que** le conservateur est choisi parmi l'acide sorbique, le sorbate de sodium, le sorbate de potassium, le sorbate de calcium, le parahydroxybenzoate d'hepthyle, l'acide benzoïque, le benzoate de sodium, le benzoate de potassium, le benzoate de calcium, le chlorure de benzalkonium, l'acide formique, le formiate de sodium, le formiate de calcium, le formaldéhyde, l'acide acétique, les acétates de potassium, l'acétate de sodium, le diacétate de sodium, l'acétate de calcium, l'acétate d'ammonium, l'acide déhydroacétique, le déhydroacétate de sodium, l'acide lactique, l'acide propanoïque, l'acide borique, l'acide malique, l'acide fumarique ou une combinaison de ces conservateurs, préférentiellement, le chlorure de benzalkonium.

9. Composition pour utilisation selon l'une des revendications précédentes, **caractérisée en ce qu'**elle comprend en outre un agent anti-bactérien, un agent antifongique, un agent anti-douleur, un agent anti-inflammatoire, un agent hydratant, des vitamines, ou un mélange de ces composés.

## Patentansprüche

1. Eine Rezeptur zur Anwendung auf der Schleimhaut als Nasenlösung zur Vorbeugung gegen und Behandlung von winterliche(n) Erkrankungen, insbesondere der oberen Atemwege, die mindestens einen der die Schleimhaut mit einer Schicht überziehenden Wirkstoffe Maltit, Isomaltit, Mannit, Isosorbid, Glycol, Erythit, Arabit, Ribit, Galaktit, Volemitol, Lactit oder eine Kombination dieser Inhaltsstoffe enthält, zudem als Feuchthaltemittel mindestens eine der Verbindungen Calciumacetat, Glycerin, 1,2-Propandiol, Honig, NatriumCarboxymethylcellulose, Magnesiumoxid, Sorbit, Calciumchlorid, Fructose, Glucose, Maltose, Mannose, Polyethylenglycol, Triacetin, Natriummalat, Milchsäure, Phosphate und mindestens einen Säureregulator zur Einstellung des pH-Werts auf 3,3 bis 4, vorzugsweise von 3,5 bis 3,7.

2. Eine Rezeptur gemäß Anspruch 1, die durch einen Massenanteil der die Schleimhaut überziehenden Wirkstoffe an der Gesamtmasse der Rezeptur von 5 bis 25 Prozent, vorzugsweise 10 bis 20 Prozent, gekennzeichnet ist.

3. Eine Rezeptur gemäß einem der Ansprüche 1 und 2, die **dadurch gekennzeichnet ist, dass** der die Schleimhaut überziehende Wirkstoff ein Maltitsirup ist.

4. Eine Rezeptur gemäß einem der Ansprüche 1 bis 3, die **dadurch gekennzeichnet ist, dass** das Feuchthaltemittel einen Massenanteil an der Gesamtmasse der Rezeptur von 0,1 bis 5 Prozent, vorzugsweise 0,5 bis 2 Prozent, darstellt.

5. Eine Rezeptur gemäß einem der Ansprüche 1 bis 4, der **dadurch gekennzeichnet ist, dass** das Feuchthaltemittel Glycerin ist.

6. Eine Rezeptur gemäß einem der Ansprüche 1 bis 5, die **dadurch gekennzeichnet ist, dass** als Säureregulator eines der Paare Zitronensäure/Citrat, Phosphorsäure/Phosphat, Weinsäure/Tartrat, Äpfelsäure/Malat, Sorbinsäure/Sorbat verwendet wird, vorzugsweise eine Kombination aus Zitronensäure und Trinatriumcitrat-Dihydrat.

7. Eine Rezeptur gemäß den Ansprüchen 1 bis 6, die **dadurch gekennzeichnet ist, dass** sie zusätzlich mindestens einen Konservierungsstoff und mindestens einen Geschmacksstoff enthält.

8. Eine Rezeptur gemäß den vorstehenden Ansprüchen, die **dadurch gekennzeichnet ist, dass** das Konservierungsmittel einer der folgenden Stoffe oder eine Kombination davon ist: Sorbinsäure, Natriumsorbat, Kaliumsorbat, Calciumsorbat, Heptylparaben, Benzoesäure, Natriumbenzoat, Kaliumbenzoat, Calciumbenzoat, Benzalkoniumchlorid, Ameisensäure, Natriumformiat, Calciumformiat, Formaldehyd, Essigsäure, Kaliumacetat, Natriumacetat, Natriumdiacetat, Calciumacetat, Ammoniumacetat, Dehydracetsäure, Natriumdehydracetat, Milchsäure, Propionsäure, Borsäure, Äpfelsäure, Fumarsäure - vorzugsweise Benzalkoniumchlorid.

9. Eine Rezeptur gemäß einem der vorstehenden Ansprüche, die **dadurch gekennzeichnet ist, dass** sie zusätzlich einen antibakteriellen Wirkstoff, einen antimykotischen Wirkstoff, ein Schmerzmittel, einen entzündungshemmenden Wirkstoff, einen hydratisierenden Wirkstoff, Vitamine oder eine Mischung dieser Bestandteile enthält.

## Claims

1. Composition created specifically for applying to mucous membranes in the form of a nasal solution for preventing and treating wintertime afflictions, in particular those affecting the upper respiratory tract, containing at least one mucous membrane-lining agent selected from syrups of maltitol, isomaltitol, mannitol, isosorbide, glycol, erythritol, arabitol, ribitol, dulcitol, volemitol or lactitol or a combination of these ingredients, at least one moisturising agent selected from sodium chloride, calcium acetate, glycerine, propylene glycol, honey, sodium carboxymethylcellulose, magnesium oxide, sorbitol, calcium chloride, fructose, glucose, maltose, mannose, polyethylene glycols, triacetine, sodium malate, lactic acid or phosphates and at least one pH adjuster, with the said composition having a pH between 3.3 and 4, preferably between 3.5 and 3.7.

2. Composition selected according to claim 1, specifically with a membrane-lining agent content between 5% and 25% by weight, preferably between 10% and 20%, compared with the total weight of the composition.

3. Composition selected according to one of claims 1 and 2, where the agent lining the mucous membranes is a maltitol syrup.

4. Composition selected according to one of claims 1 to 3, where the moisturising agent content is between 0.1% and 5% by weight, preferably between 0.5% and 2%, compared with the total weight of the composition.

5. Composition selected according to one of claims 1 to 4, where the moisturising agent is glycerine.

6. Composition selected according to one of claims 1 to 5, with the pH adjuster being chosen among the following pairs - citric acid/citrate, phosphoric acid/phosphate, tartaric acid/tartrate, malic acid/malate, sorbic acid/sorbate, with a preference for a combination of citric acid and trisodium citrate dihydrate.

7. Composition selected according to one of claims 1 to 6, which contains in addition at least a preservative and a fragrance.

8. Composition selected according to one of the aforementioned claims, in which the preservative is chosen from among the following - sorbic acid, sodium sorbate, potassium sorbate, calcium sorbate, ethyl parahydroxybenzoate, benzoic acid, sodium benzoate, potassium benzoate, calcium benzoate, benzalkonium chloride, formic acid, sodium formiate, calcium formiate, formaldehyde, acetic acid, potassium acetates, sodium acetate, sodium diacetate, calcium acetate, ammonium acetate, dehydroacetic acid, sodium dehydroacetate, lactic acid, propanoic acid, boric acid, malic acid, fumaric acid or any combination of such preservatives, with a preference for benzalkonium chloride.

9. Composition selected according to one of the aforementioned claims, containing, in addition, an anti-bacterial agent, an anti-fungal agent, a pain-relieving agent, an antiinflammatory agent, a moisturising agent, vitamins, or a mixture of such components.
